(19) Europäisches Patentamt
European Patent Office
Office européen des brevets

(11) Publication number: **0 545 543 A2**

(12) **EUROPEAN PATENT APPLICATION**

(21) Application number: **92309940.2**

(22) Date of filing: **29.10.92**

(51) Int. Cl.$^5$: **C07C 251/22,** B41M 5/26

(30) Priority: **31.10.91 JP 286093/91**

(43) Date of publication of application: **09.06.93 Bulletin 93/23**

(84) Designated Contracting States: **DE FR GB**

(71) Applicant: **MITSUI TOATSU CHEMICALS, Inc.**
**2-5 Kasumigaseki 3-chome**
**Chiyoda-Ku Tokyo 100 (JP)**

(72) Inventor: **Ohyama, Tsukasa**
**2-308-321 Kamishirakawamachi**
**Ohmuta-shi, Fukuoka (JP)**
Inventor: **Shimokawa, Yasushi**
**2-308-725 Kamishirakawamachi**
**Ohmuta-shi, Fukuoka (JP)**
Inventor: **Ghoda, Isamu**
**7-3-30 Kasamatsudoori, Hyogo-ku**
**Kobe-shi, Hyogo (JP)**
Inventor: **Koshida, Hitoshi**
**12-5-510 Koshienguchi Kitamachi**
**Nishinomiya-shi, Hyogo (JP)**
Inventor: **Takuma, Keisuke**
**300-424 Hirabarumachi**
**Ohmuta-shi, Fukuoka (JP)**
Inventor: **Matsuzaki, Yoriaki**
**2-13-501 Koganemachi**
**Ohmuta-shi, Fukuoka (JP)**

(74) Representative: **Paget, Hugh Charles Edward et al**
**MEWBURN ELLIS 2 Cursitor Street**
**London EC4A 1BQ (GB)**

(54) **Indoaniline compounds and their use in heat-sensitive transfer recording compositions.**

(57) Described herein are an indoaniline compound represented by the following formula (1):

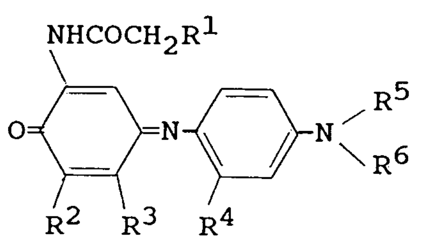

(1)

wherein $R^1$ represents an aryl or aryloxy group, $R^2$, $R^3$ and $R^4$ each represents a hydrogen or halogen atom or a $C_{1-4}$ alkyl group, and $R^5$ and $R^6$ each represents a $C_{1-4}$ alkyl group; use of the compound as a heat-sensitive transfer recording dye; an ink composition for a transfer sheet, said composition containing the dye, a binder resin, and an organic solvent and/or water; and a transfer sheet comprising a base sheet and a dye-carrying layer formed on one side of the base sheet, said layer containing therein the above dye.

EP 0 545 543 A2

Background of the Invention

1) Field of the Invention

This invention relates to indoaniline compounds and use thereof, namely, heat-sensitive transfer recording dyes (hereinafter abbreviated as "sublimable dyes") in color hard copies by a thermal, sublimation transfer recording process, heat-sensitive transfer recording ink compositions and transfer sheets.

2) Description of the Related Art

The thermal transfer process making use of a sublimable dye is one of thermal transfer printing processes, which comprises coating a thin capacitor paper sheet or PET film of several micrometers with a sublimable dye in the form of an ink composition and then selectively heating the thus-coated sheet or film by a thermal head to transfer the sublimable dye onto a recording paper sheet. The thermal transfer process is now used as a method for recording various image information to provide tangible images, i.e., hard copies.

Sublimable dyes usable in the thermal transfer process are required to have characteristic features including the availability in a wide variety of colors, excellent color mixability, strong dyeing power and relatively high stability. The thermal, sublimation transfer recording process has a unique characteristic feature not found in the other photographic printing processes in that the amount of a dye to be sublimed is dependent on thermal energy and the color density available after dyeing can be controlled in an analog manner.

Indoaniline compounds have attracted particular interests as sublimation-transferable cyan dyes (Japanese Patent Laid-Open Nos. 22993/1986 and 31292/1986). Only an extremely small fraction of the indoaniline compounds, however, have a high sublimation velocity and can provide a stable picture after transfer. There is hence a long standing desire for the development of a dye capable of fully meeting the above-desired requirements for sublimable dyes.

SUMMARY OF THE INVENTION

With a view toward overcoming the problems described above, the present inventors have conducted an extensive investigation. As a result, it has been found that the sublimation velocity of a heat-sensitive transfer recording dye at the time of its transfer is generally governed by both intermolecular action of the dye and interaction between dye molecules and an ink-forming binder resin.

It has become clear that to be an excellent dye, the sublimable dye has good solubility in an ink solvent, a lower melting point, and interaction with an ink-forming binder resin sufficiently small to avoid impairment to the storage stability after the production of transfer sheets. It has therefore been found that indoaniline compounds represented by the below-described formula (1) can be used as excellent heat-sensitive sublimation transfer cyan dyes, leading to the completion of the present invention.

An object of the present invention is to provide an indoaniline compound and use thereof, namely, a heat-sensitive transfer recording dye having a high sublimation velocity and capable of providing stable picture after transfer, a heat-sensitive transfer recording ink composition and a transfer sheet.

The present invention, therefore, provides an indoaniline compound represented by the following formula (1):

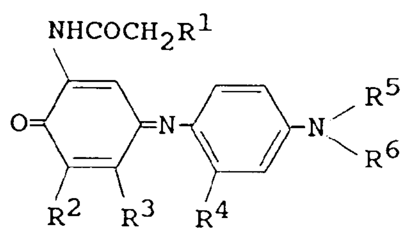

(1)

wherein $R^1$ represents an aryl or aryloxy group, $R^2$, $R^3$ and $R^4$ each represents a hydrogen or halogen atom or a $C_{1-4}$ alkyl group, and $R^5$ and $R^6$ each represents a $C_{1-4}$ alkyl group; as well as use thereof, that is, a heat-sensitive transfer recording dye using the compound, a heat-sensitive transfer recording ink composition containing the dye, a binder resin and an organic solvent and/or water, and a transfer sheet composed of a base

sheet and a dye-carrying layer formed on one side of the base sheet, said dye-carrying layer containing the above dye therein.

DETAILED DESCRIPTION OF THE INVENTION

Indoaniline compounds according to the present invention are those represented above by the formula (1).

In the formula (1), illustrative of $R_1$ include aryl groups such as phenyl, p-tolyl, m-tolyl, o-tolyl, p-chlorophenyl, m-chlorophenyl and o-chlorophenyl groups; and aryloxy groups such as phenoxy, p-methylphenoxy, m-methylphenoxy, o-methylphenoxy, p-chlorophenoxy, p-ethylphenoxy and p-propylphenoxy groups.

Examples of $R^2$, $R^3$ and $R^4$ include a hydrogen atom, $C_{1-4}$ alkyl groups such as methyl, ethyl, n-propyl, iso-propyl, n-butyl, iso-butyl and sec-butyl groups, and halogen atoms such as fluorine, chlorine and bromine.

Examples of $R^5$ and $R^6$ include $C_{1-4}$ alkyl groups such as methyl, ethyl, n-propyl, iso-propyl, n-butyl, iso-butyl, sec-butyl and tert-butyl groups.

Each indoaniline compound of the present invention represented by the formula (1) can, when used as a dye, meet all the various requirements described above and can provide a relatively good sublimation velocity.

Preparation of each compound of the present invention represented by the formula (1) can be conducted by oxidative coupling of its corresponding substituted aniline and substituted phenol in a manner known *per se* in the art.

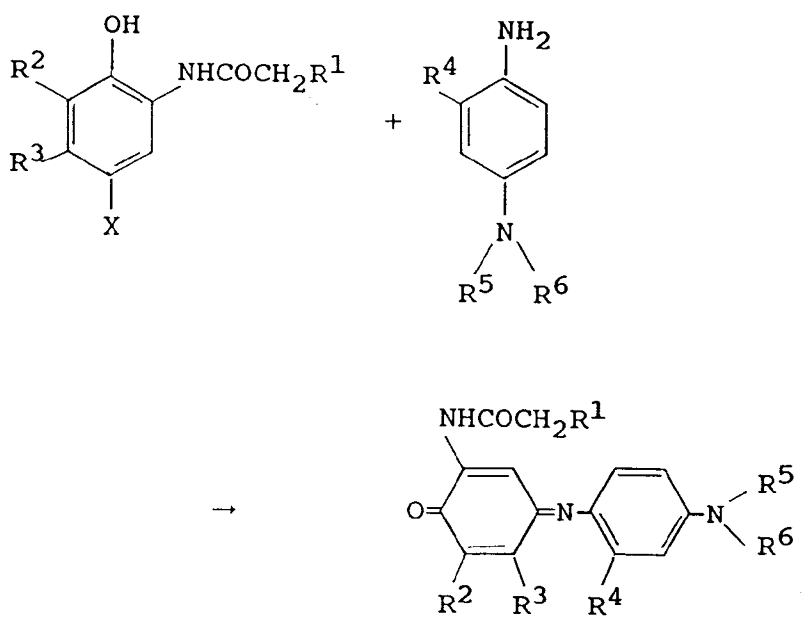

(X is an eliminative group such as H or a halogen).

Thermal transfer recording using the above compound can be carried out, for example, by formulating a heat-sensitive transfer ink composition of the above compound, coating an appropriate base material with the above-formulated ink to prepare a transfer sheet, bringing the transfer sheet into a contiguous relationship with a material on which a record is to be formed (hereinafter called a "recording material"), and then applying heat and pressure by a thermal recording head from the back side of the sheet. This causes transfer of the indoaniline compound (dye) from the sheet onto the recording material.

To formulate the heat-sensitive transfer recording ink composition of this invention, it is only necessary to mix the indoaniline compound with a suitable resin, an appropriate solvent and the like into such a recording ink. In this case, the heat-sensitive transfer recording ink generally contains the indoaniline compound in an amount of 2-5 wt.%

As a binder resin for formulating the ink described above, any resin employed in conventional printing inks can be used. Illustrative usable resins include oil-soluble resins such as rosin resins, phenol resins, xylene resins, petroleum resins, vinyl resins, polyamide resins, alkyd resins, nitrocellulose resins and alkylcellulose resins; and water-soluble resins such as maleic resins, acrylic resins, casein, shellac and animal glue.

Exemplary solvents suitable for use upon formulation of the ink include alcohols such as methanol, ethanol, propanol and butanol; cellosolves such as methylcellosolve and ethylcellosolve; aromatic solvents such as benzene, toluene and xylene; esters such as ethyl acetate and butyl acetate; ketones such as acetone, methyl ethyl ketone and cyclohexanone; hydrocarbons such as ligroin, cyclohexane and kerosine; and dimethylformamide. When a water-soluble resin is used, water can be employed either singly or in combination with one or more of the above-described solvents.

Suitable examples of the base material coated with the ink include thin paper sheets such as capacitor paper and glassine paper as well as plastic films having good heat resistance such as polyester, polyamide and polyimide films. Suitably, these base materials have a thickness on the order of 5-50 μm to improve the efficiency of heat transfer from a thermal recording head to a dye. By coating one side of the base material with an ink, a transfer sheet having a dye-carrying layer on that side can be obtained.

Examples of recording materials include fibers, woven fabrics, films, sheets, other molded or extruded articles, which can be made of polyolefin resins such as polyethylene and polypropylene; halogenated polymers such as polyvinyl chloride and polyvinylidene chloride; vinyl polymers such as polyvinyl alcohol, polyvinyl acetate and polyacrylic esters; polyester resins such as polyethylene terephthalate and polybutylene terephthalate; polystyrene resins; polyamide resins; copolymer resins, e.g., those of olefins - such as ethylene and propylene - and other vinyl monomers; ionomers; cellulose resins such as cellulose diacetate and cellulose triacetate; polycarbonates; polysulfons; polyimides; and the like. Particularly preferred are woven fabrics, sheets and films, which are made of polyethylene terephthalate.

On the other hand, general-purpose paper sheets either coated or impregnated with a mixture of a resin and fine acidic particles such as silica gel, laminates of resin films, specially converted, i.e., acetylated paper sheets, or the like can be used as recording materials. These recording materials can provide good records excellent in picture stability at elevated temperatures and humidities. Further, films of various resins and converted paper sheets made of such films can also be used.

The color production of the dye can be improved to enhance the storage stability of records by pressing and laminating, for example, a polyester film under heat on a transfer-recorded surface subsequent to the transfer recording.

Each compound of the present invention represented by the formula (1), when used as a cyan dye, permits easy tonal recording and is therefore suited for use in full-color recording, because the rate of sublimation transfer of the cyan dye can be controlled by varying the energy to be applied to a thermal head at the time of thermal transfer. It is also stable to heat, light, moisture, chemicals and the like, so that it is not subjected to thermal decomposition during transfer recording and the resulting record enjoys excellent storability.

In addition, the compounds of the present invention have good solubility in organic solvents and good dispersibility in water. It is therefore easy to formulate them into high-concentration inks in which they are evenly dissolved or dispersed. As a result, records having good color density can be obtained. The dyes of the present invention are hence valuable from the practical viewpoint.

The present invention will hereinafter be described in detail by the following examples, in which all designations of "part" or "parts" and "%" mean part or parts by weight and wt.% unless otherwise specifically indicated.

## Example 1

In 120 parts of methanol, 10 parts of 4-N,N-diethylamino -2-toluidine and 12 parts of 2-phenoxyacetylaminophenol were dissolved. To the resulting solution, 27 parts of 25% aqueous ammonia were added. While the liquid temperature was maintained at 20°C, 25 parts of ammonium persulfate were added over about 1 hour. They were then reacted for 2 hours at room temperature. After the reaction, the precipitate was collected and washed thoroughly with water, and then purified by column chromatography so that the target compound (A) represented by the following formula:

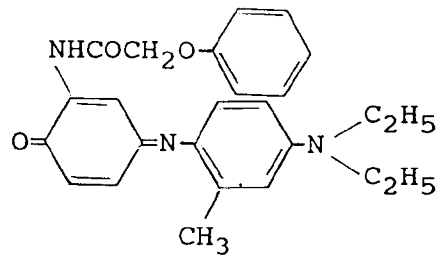

(A)

was obtained. Using the compound (A), an ink, a transfer sheet and a recording material were also prepared. Transfer recording was then performed.

Incidentally, the wavelength at the maximum absorption, λmax, of the compound in toluene was 629 nm.
Melting point: 136.1-137.4°C
Elemental analysis data ($C_{25}H_{27}N_3O_3$):

| | C | H | N |
|---|---|---|---|
| Calculated (%) | 71.92 | 6.52 | 10.06 |
| Found (%) | 71.85 | 6.55 | 10.03 |

(1) Formulation of the ink

| | |
|---|---|
| Compound of the formula (A) | 3 parts |
| Polybutyral resin | 4.5 parts |
| Methyl ethyl ketone | 46.25 parts |
| Toluene | 46.25 parts |

The ink was formulated by mixing a dye mixture of the above composition for about 30 minutes in a paint conditioner while using glass beads.

(2) Preparation method of the transfer sheet

Using a gravure proof press (groove depth: 30 μm), the above ink was coated on a 9-μm thick polyethylene terephthalate film, which had been subjected to heat-resisting treatment on the back side thereof, to give a dry coat weight of 1.0 g/m$^2$. The film so coated was then dried.

(3) Preparation of the recording material

| | |
|---|---|
| Polyester resin ("VYLON 103", trade name; product of Toyobo Co., Ltd.; Tg: 47°C) | 0.8 part |
| EVA type, high-molecular plasticizer ("ELVALOY 741P", trade name; product of Mitsui Polychemical, Inc.; Tg: 37°C) | 0.2 part |
| Amino-modified silicone ("KF-857", trade name; product of Shin-Etsu Chemical Co., Ltd.) | 0.04 part |
| Epoxy-modified silicone ("KF-103", trade name; product of Shin-Etsu Chemical Co., Ltd.) | 0.04 part |
| Methyl ethyl ketone/toluene/cyclohexane (weight ratio: 4:4:2) | 9.0 parts |

The above components were mixed to prepare a coating formulation. A synthetic paper web ("YUPO FPG#150", trade name; product of Oji-Yuka Synthetic Paper Co., Ltd.) was coated with the coating formulation by a bar coater (Model No. 1; manufactured by RK Print Coat Instruments Company) to give a dry coat weight of 4.5 g/m$^2$, followed by drying at 100°C for 15 minutes.

(4) Transfer recording

The above transfer sheet and the recording material were brought into a contiguous relationship with the ink-coated side and the formulation-coated side facing each other. From the back side of the transfer sheet, recording was performed under the conditions of 10 V thermal head application voltage and 4.0 msec printing time, whereby a cyan-colored record having a color density of 2.85 was obtained. Owing to the high compatibility of the compound (A) with the binder resin, the record was uniform and clear. Incidentally, the color density was measured using a densitometer "Model RD-514" (filter: Wratten No. 58) manufactured by Macbeth Company, U.S.A.

The color density was calculated in accordance with the following equation:

$$\text{Color density} = \log_{10} (I_o/I)$$

where

$I_o$: Intensity of light reflected from a standard white reflector plate, and

I: Intensity of light reflected from the sample object.

A light resistance test of the record thus obtained was also conducted at a black panel temperature of 63 ± 2°C by using a xenon fadeometer manufactured by Suga Testing Machines Co., Ltd. No substantial color variation was observed after 40-hour exposure. The stability of the picture was excellent even at the elevated

temperature and humidity.

In addition, the fastness of the recorded image was judged based on the degree of sharpness of the recorded image after the image was left over for 48 hours in an atmosphere of 50°C and also on the extent of smear of a white paper sheet when the recorded surface was rubbed with the white paper sheet. The sharpness of the image remained unchanged and the white paper sheet was not smeared, whereby the fastness of the recorded image was good.

Furthermore, a high-concentration ink, which is required for high-density transfer in the fabrication of OHP sheets or the like, was successfully prepared with ease because of the substantially high solubility of the compound (A) in the ink. Use of the transfer sheet coated with the above ink successfully provided high-concentration OHP sheets.

Example 2

In a liquid mixture consisting of 63 parts of methanol and 38 parts of water, 11 parts of 4-N,N-diethylphenylenediamine and 13 parts of 2-phenoxyacetylamino-3-cresol were dissolved, followed by the addition of 31 parts of 25% aqueous ammonia. While the temperature of the resulting liquid mixture was maintained at 20°C, 76.6 parts of a 25% aqueous solution of silver nitrate were added dropwise over about 1 hour. They were then reacted for 2 hours at room temperature. After the reaction, the reaction mixture was extracted with chloroform. The chloroform layer was thoroughly washed with water and then purified by column chromatography, so that the target compound (B) represented by the following formula:

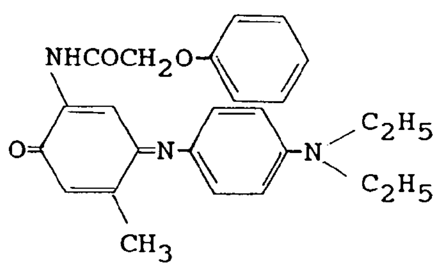

(B)

was obtained.

The wavelength at the maximum absorption, λmax, of the compound in chloroform was 629 nm.

Melting point: 141.2-142.5°C

Elemental analysis data ($C_{25}H_{27}N_3O_3$):

| | C | H | N |
|---|---|---|---|
| Calculated (%) | 71.92 | 6.52 | 10.06 |
| Found (%) | 71.97 | 6.50 | 10.11 |

Similarly to Example 1, an ink was formulated, a transfer sheet and a recording material were prepared, and transfer recording was performed, whereby a cyan-colored record having a color density of 2.85 was obtained. The compound (B) had high compatibility with the binder resin so that the recorded image was uniform in density and was sharp in definition.

The record was subjected to a light resistance test in a similar manner to Example 1. The record did not undergo any substantial variations, so that the image had excellent stability at the elevated temperature and humidity. In addition, a fastness test was conducted in a similar manner to Example 1. The sharpness of the image remained unchanged and the white paper was not smeared. The fastness of the recorded image was therefore good. Furthermore, the solubility of the compound (B) in the ink was substantially high so that a high-concentration ink, which is required for high-density transfer in the fabrication of OHP sheets or the like, was successfully prepared with ease. When transfer was conducted using the transfer sheet containing the ink, high-density OHP sheets were successfully obtained.

Examples 3-11

Following the procedures of Example 1, the cyan dyes shown in Table 1 were prepared. Similarly to Example 1, inks were formulated, transfer sheets and recording materials were prepared, and transfer recording was performed. The various records shown in Table 1 were obtained. The cyan dyes shown in Table 1 each has a high compatibility with the binder resin so that the image obtained were uniform in density and sharp in definition.

Those records were all subjected to a light resistance test in a similar manner to Example 1. Those records did not undergo any substantial variations, so that the image showed excellent stability at the elevated temperature and humidity. In addition, a fastness test was also conducted in a similar manner to Example 1. The sharpness of the image remained unchanged and the white paper sheets were not smeared. The fastness of the recorded image was therefore good. Furthermore, the solubility of each compound in the ink was substantially high so that a high-concentration ink, which is required for high-density transfer in the fabrication of OHP sheets or the like, were successfully prepared with ease. When transfer was conducted using the transfer sheets coated with the inks, high-concentration OHP sheets were successfully obtained.

In Table 1, the light resistance and fastness of the recorded image are each ranked "A", when they were rated good as a result of judgement.

Table 1

| Ex. | Structural formula | Maximum optical density | Light resistance | Fastness of the recorded image |
|---|---|---|---|---|
| 3 | 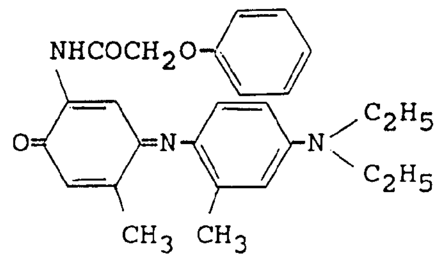 | 2.80 | A | A |
| 4 | 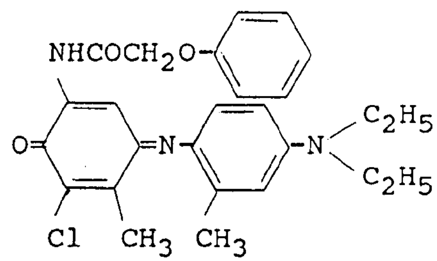 | 2.35 | A | A |
| 5 | 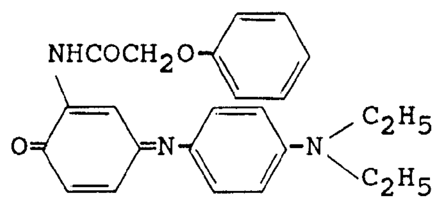 | 2.83 | A | A |

Table 1 (Cont'd)

| Ex. | Structural formula | Maximum optical density | Light resistance | Fastness of the recorded image |
|---|---|---|---|---|
| 6 | 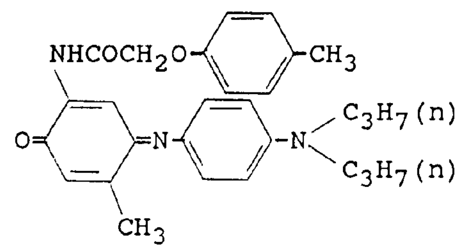 | 2.76 | A | A |
| 7 | 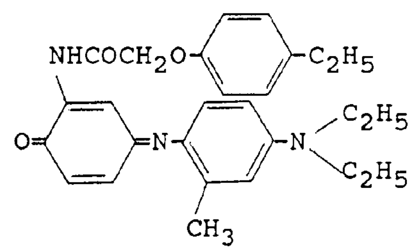 | 2.74 | A | A |
| 8 | 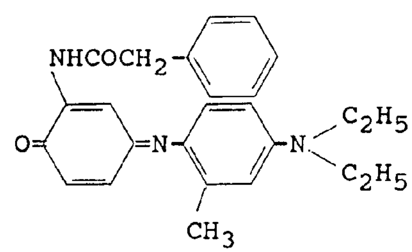 | 2.80 | A | A |

Table 1 (Cont'd)

| Ex. | Structural formula | Maximum optical density | Light resistance | Fastness of the recorded image |
|---|---|---|---|---|
| 9 | 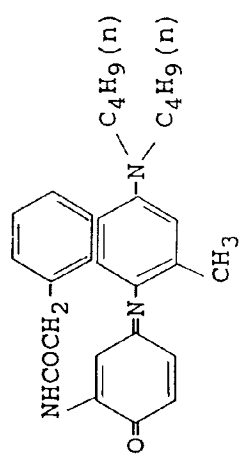 | 2.70 | A | A |
| 10 | 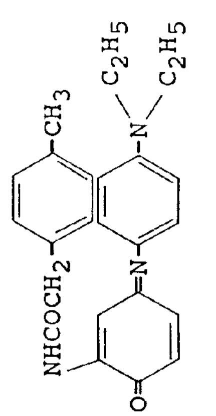 | 2.70 | A | A |
| 11 | 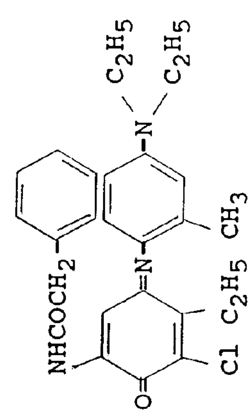 | 2.25 | A | A |

## Comparative Examples

In each comparative example, an ink was formulated using the dye shown in Table 2, transfer sheets and recording materials were prepared, and transfer recording was performed, as in Example 1. In addition, in a

similar manner to Example 1, a light resistance test and a fastness test were conducted. As a result, it was impossible to obtain any record which was satisfactory in all the requirements of maximum optical density, light resistance and the fastness of the recorded image. Furthermore, the dye had poor compatibility with the binder resin so that the image obtained was not sharp in definition and the white paper sheet was smeared substantially. The record was hence poor.

Besides, due to the low solubility of the dye in the ink, the dye underwent significant precipitation upon preparation of the high-concentration ink, thereby making it impossible to prepare any high-density sheet.

In Table 2, the light resistance and the fastness of the recorded image are each ranked "A", when they were rated good as a result of judgement. On the other hand, they are each ranked "C", when rated poor.

Table 2

| Comp. Ex. | Structural formula | Maximum optical density | Light resistance | Fastness of the recorded image |
|---|---|---|---|---|
| 1 | 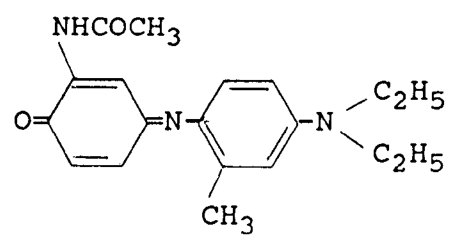 | 2.2 | A | C |
| 2 | 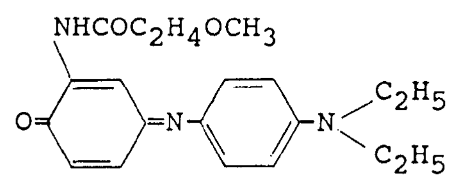 | 2.3 | C | C |
| 3 | 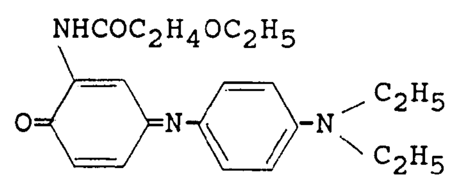 | 2.3 | C | C |

Table 2 (Cont'd)

| Comp. Ex. | Structural formula | Maximum optical density | Light resistance | Fastness of the recorded image |
|---|---|---|---|---|
| 4 | 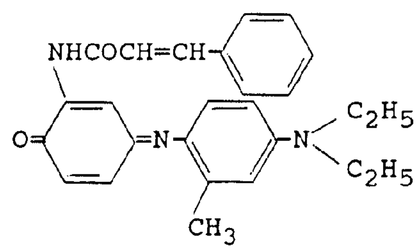 | 2.1 | A | C |
| 5 | 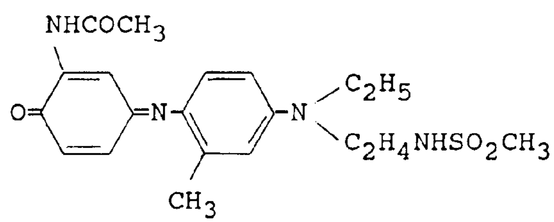 | 1.3 | C | A |
| 6 | 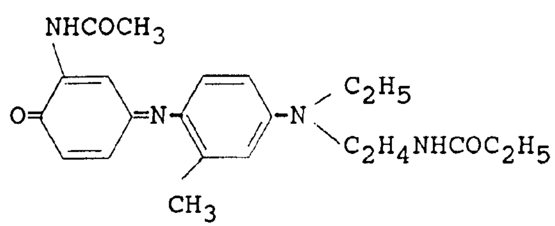 | 1.6 | C | A |

## Claims

1. An indoaniline compound represented by the following formula (1):

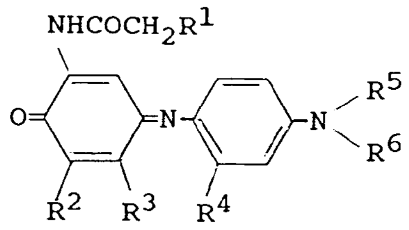

(1)

wherein $R^1$ represents an aryl or aryloxy group, $R^2$, $R^3$ and $R^4$ each represents a hydrogen or halogen atom or $C_{1-4}$ alkyl group, and $R^5$ and $R^6$ each represents a $C_{1-4}$ alkyl group.

2. Use of the indoaniline compound of claim 1 as a heat-sensitive transfer recording dye.

3. A heat-sensitive transfer recording ink composition comprising the compound of claim 1, a binder resin and an organic solvent and/or water.

4. A transfer sheet comprising a base sheet and a dye-carrying layer formed on one side of the base sheet, said layer containing therein the compound of claim 1.